# EUROPEAN PATENT SPECIFICATION

(11) **EP 4 436 537 B1**
(45) Date of publication and mention of the grant of the patent: **14.05.2025**
(21) Application number: 22801363.7
(22) Date of filing: 05.10.2022
(51) Int. Cl.: A61K 8/31, A61K 8/37, A61K 8/73, A61Q 5/12

(54) **HAIR CONDITIONING COMPOSITION COMPRISING PARTICULATED STARCH**
HAARPFLEGEZUSAMMENSETZUNG MIT TEILCHENFÖRMIGER STÄRKE
COMPOSITION DE CONDITIONNEMENT CAPILLAIRE COMPRENANT DE L'AMIDON PARTICULAIRE

(30) Priority: 27.10.2021 EP 21205129
(43) Date of publication of application: 02.10.2024
(73) Proprietor: Unilever IP Holdings B.V., 3013 AL Rotterdam (NL); Unilever Global IP Limited, Wirral, Merseyside CH62 4ZD (GB)
(72) Inventor: AVERY, Andrew Richard, 6708 WH Wageningen (NL); ORTUOSTE ELCORO, Nerea, 6708 WH Wageningen (NL)
(74) Representative: Chisem, Janet
(86) International application number: PCT/EP2022/077657
(87) International publication number: WO 2023/072537

(56) References cited:
- EP-A1- 1 927 346
- CN-A- 113 274 338
- US-A1- 2020 368 135
- AGRANA STARCH: "AGRANA STARCH EO-free Vitamin Shampoo with SynCrystal Ivory +CORN PO4 PH "B"", ECKART VITAMIN BOMB FRAGRANCE 0,20 BELL EUROPE EUXYL K712 SODIUM BENZOATE (AND) POTASSIUM SORBATE 0, 5 April 2012 (2012-04-05), pages 1 - 1, XP055915957, Retrieved from the Internet <URL::https://www.agrana.com/fileadmin/inhalte/agrana_group/AGRANA_Starke/Kosmetikformulierungen/Men_Care/AGRANA_-_EO-free_Vitamin_Shampoo_with_SynCrystal_Ivory_and_CORN_PO4_PH_B.pd> [retrieved on 20220427]
- AGRANA: "AGRANA STARCH GRANULAR STARCHES for EMULSIONS and ANHYDROUS CREAMS", 1 March 2016 (2016-03-01), XP055915736, Retrieved from the Internet <URL::https://www.ulprospector.com/documents/1399420.pdf?bs=17598&b=443320&st=20&r=eu&ind=personalcare> [retrieved on 20220426]
- AGRANA: "Dry Shampoo Mousse with RICE PO4 NATURAL", 12 June 2018 (2018-06-12), pages 1 - 1, XP055916694, Retrieved from the Internet <URL:: https : //www. agrana. com/fileadmin/inhalte/agrana_group/2017/images/Starch/Folder/Kosmetik/Formulierungen/AGRANA_-_Dry_Shampoo_Mousse_with_RICE_NS.pdf> [retrieved on 20220429]

## Description

### Field of the Invention

The invention lies in the field of hair conditioner compositions that deliver conditioning benefits to hair, without the inclusion of silicone.

### Background of the Invention

Consumers desire hair treatments, such as shampoos and conditioners, that provide conditioning benefits, for example smoothness, ease of comb and softness to their hair.

Conditioning benefits are desired and perceived by the consumer both during and after the wash process. Thus, conditioning is evaluated both when the hair is wet and when it is dry. As such, it is desirable that rinse-off haircare products such as conditioners provide multiple benefits at different stages of use. Wet conditioning and dry conditioning are different benefits and are typically delivered in different ways.

The impact of the gel phase component of a conditioning product is most apparent when wet or damp hair is evaluated, as well as during application and distribution through the hair.

Product rheology is a key attribute to consumers. Conditioners having superior rheology, such as thickness and yield stress can provide improved conditioning benefits and are preferred by consumers.

Silicones are primarily used to provide conditioning benefits at the post drying stages. In silicone-free conditioner formulations alternative approaches are required to provide such benefits.

Starches have been used in hair conditioning compositions:
WO2017/172117 (L'Oreal) - discloses a composition for treating keratinous substrates, comprising (a) a cationic agent comprising a defined first quaternary ammonium compound, a defined second quaternary compounds of imidazoline; (b) a modified starch; (c) a defined first silane compound, (d) a second silane compound, (e) at least one cationic vinylpyrrolidone polymer and (f) water, to provide conditioning and styling benefits. Hydroxypropyl starch phosphates are preferred and exemplified.
US2006/0182702 (L'Oreal) - uses a composition comprising, a cationic surfactant, a starch which is preferably modified, a non-silicone cationic polymer with a cationic charge density of greater than or equal to 5 meq/g, and a nonionic non-polymeric solid compound with a melting point of greater than or equal to 35 degrees C and/or with a viscosity, at a temperature of 40 degrees C and at a shear rate of 1 s⁻¹, of greater than or equal to 1 Pa.s, to provide an improved conditioning effect to hair, especially smoothing of the ends.
FR2976488 (L'Oreal) - discloses a cosmetic composition comprising a combination of pumice particles (i); one or more starches (ii); one or more solid fatty alcohols (iii); and one or more fatty esters (iv) to provide durable treatment of keratin fibers that are damaged on the surface. A use for smoothing hair is disclosed. Distarch phosphate of pregelatinized hydroxypropyl maize is exemplified.
US2005069511 (L'Oreal) discloses a cosmetic composition, comprising, at least one starch, at least one carboxylic ester, water and up to 20 wt % of fatty phase relative to the total weight of composition. The use of these compositions, based on a specific ester and a starch allegedly provides hair that is easy to disentangle and is smooth from root to tip, with an improvement in the hold of the style. A pregelatinized corn distarch phosphate or Potato fecula modified with 2-chloro-ethylamidodipropionic acid neutralized with sodium hydroxide are utilised in the examples on wet hair to provide wet hair that is not heavy and hair shaping is easy.
US2016038397- (Penford Corp) dislcoses a cosmetic composition comprising: water; an ingredient selected from detergents and non-detersive conditioning agents; a cationic starch characterized by: a) an amylopectin/amylose weight ratio of greater than or equal to 60/40; b) an apparent cationic molecular weight of greater than or equal to 12 million daltons; and c) a cationic degree of substitution of from 0.5 meq/g to 2.5 meq/g to provide improved conditioning properties to personal care compositions. The personal care formulations can be used with or without silicone. Shampoos comprising cationic substituted starches allegedly give conditioning benefits in the wet stage.
EP1927346 discloses aqueous compositions for keratin fibres comprising an oil and/or wax, a natural starch, an emulsifier and a film forming polymer.

Despite the prior art, there remains a need to provide conditioning benefits, during a wash process, that are apparent in the dry stage without compromising on consumer desired viscosity characteristics.

We have now found that improved dry conditioning can be delivered from a conditioner by the use of a particulate starch, blended with an oil, along with a surprising rheological benefit reflected in superior yield stress.

### Definition of the Invention

In a first aspect, the invention provides a hair conditioner composition comprising
A) 0.01 to 5 wt % (by weight of the total composition) of a blend comprising
   i) starch particles having a Dv(50) particle size of from 1 to 12 microns; and
   ii) a hydrophobic non-silicone oil;
   in a weight ratio of starch to oil of from 0.5:1 to 1: 0.5;
B) 0.01 to 5 wt % of an emulsified non-silicone oil;
wherein A) and B) are dispersed within:
C) a conditioning gel phase comprising a cationic surfactant and a fatty alcohol; and
wherein the conditioner is free from silicone.

In a second aspect, there is provided a method of treating hair, comprising the step of applying to hair a composition of the first aspect.

Use is also provided of a blend of particulate starch particles and non-silicone oil in a silicone free composition of the first aspect to provide conditioning benefits to hair.

In the use of the invention, the conditioning benefit is preferably a reduced coefficient of friction compared with hair treated with the same composition comprising a non-particulate starch.

### Detailed Description of the Invention

The hair conditioner composition of the invention comprises:
A) 0.01 to 5 wt % (by weight of the total composition) of a blend comprising:
   i) a starch, which is particulate starch, having a particle size of from 1 to 12 microns; and ii) a hydrophobic non-silicone oil; in a weight ratio of starch to oil of from 0.5:1 to 1: 0.5.

The blend of starch and oil is present in an amount of from 0.01 to 5 wt %, preferably from 0.1 to 4 wt %, more preferably from 0.5 to 4 wt %, most preferably from 0.5 to 3 wt %.

The weight ratio of starch to oil in the blend, is from 0.5:1 to 1: 0.5, preferably 1:1.

### The Starch

The compositions of the invention comprise a starch, which is a particulate starch. The starch remains particulate in the composition.

The starch for use in the invention has a Dv(50) particle size of from 1 to 12 microns, preferably from 2 to 10 microns, most preferably from 3 to 9 microns.

Any suitable particle size analyser, such as a Malvern Mastersizer 3000 may be used to characterize starch particles. A suitable refractive index (RI) of starch is 1.530 (as given in Holes in Starch Granules: Confocal, SEM and Light Microscopy Studies of Starch Granule Structure, Baldwin, P.M., Adler, J., Davies, M.C., Melia, C.D., Starch/Starke 46 (1994) Nr.9, S. 341-346).

A laser diffraction technique may be employed. The sample is dispersed in water (which has a RI of 1.330) and passed into a sample window using a recirculating cell, where any particles present scatter the light. The refractive indices for both the particles and the suspending media are used for particle size determination. The following method may suitably be used:
Starch powder (0.1 g) is suspended in 10mL of deionised water and pipetted into a Mastersizer 3000 Hydro Medium Volume cell until a 5 % obscuration limit is reached. This process is preferably repeated three times for each starch sample at a stirrer speed of 2400 rpm.

Results are reported here as 'particle size (µm) against volume (%)' for Dv (10), Dv (50) and Dv (90) indicating that 10%, 50% and 90% respectively, of particles are smaller than the quoted size

The starches for use in the compositions of the invention are in the form of discrete particles. The starches for use in the present invention remain in particulate form in the compositions of the invention.

The starch is not a gelatinised or "swelling" starch. Gelatinised, or gelatinisable starches do not remain as particles when incorporated in a formulation such as a hair treatment composition. Gelatinisation depends on the hydrophilic nature of the starch. In one study, Senanayake et al (International Journal of Food Science; Volume 2014, Article ID 148982; Suraji Senanayake, Anil Gunaratne, K.K.D.S. Ranaweera and Arthur Bamunuarachchi), starches substituted with hydroxypropyl groups showed significantly higher levels of swelling power and water-soluble index compared to the unmodified native starch. According to the authors, the presence of hydroxypropyl groups, which are hydrophilic, enhances the attraction of water molecules into the granular structure that causes early swelling in the granule.

Preferably, the starch is selected from rice starch, quinoa starch, amaranth starch and mixtures thereof. More preferably, the starch is selected from rice starch, quinoa starch and mixtures thereof, most preferably the starch is rice starch.

Highly preferred rice starches may be selected from those that have been cationically modified or combined with cetrimonium chloride (for example D.S.A 7 rice starch, available from Argana Starch), crosslinked di-phosphate starches (for example Rice PO4 Natural from Agrana Starch), or a natural rice starch (for example Reisita Natural, available from Agrana Starch), and mixtures thereof.

The starch is blended with a hydrophobic non-silicone oil. The starch particle is thus within a continuous oil phase.

### The hydrophobic non-silicone oil

The oils for use in the compositions of the invention are hydrophobic non-silicone oils.

Suitable hydrophobic non-silicone oils are selected from hydrocarbon oils, fatty ester oils and mixtures thereof.

The hydrocarbon oils can be natural or synthetic.

Straight chain hydrocarbon oils will preferably contain from about 12 to about 30 carbon atoms. Also suitable are branched chain hydrocarbon oils, which preferably contain from about 12 to about 42 carbon atoms. Also suitable are polymeric hydrocarbons of alkenyl monomers, such as C2 to C6 alkenyl monomers.

Specific examples of suitable hydrocarbon oils include paraffin oil, mineral oil, polyalphaolefin, squalane, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Another suitable material is polyisobutylene.

A preferred polyalphaolefin is commercially available as Silkflo 366 ^{™} (dec-1-ene) ex Ineos. Suitable fatty esters are characterised by having at least 6 carbon atoms and include esters with hydrocarbyl chains derived from fatty acids or alcohols. Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as C1-C22 carboxylic acids. Preferred materials include cocoa butter, palm stearin, sunflower oil, soyabean oil and coconut oil.

The hydrophobic non-silicone oil is preferably selected from hydrocarbon oils selected from paraffin oil, mineral oil, polyalphaolefin oil, esters with hydrocarbyl chains derived from fatty acids or alcohols and mixtures thereof.

### The emulsified non-silicone oil

The compositions of the invention comprise an emulsified non-silicone oil. The oil is preferably described as above, for the hydrophobic non-silicone oil for use in the blend with the starch. The oil for use in the blend with the starch and the emulsified oil can be the same or different.

The emulsified non-silicone may be made by any suitable process and a skilled person in the art will know how to prepare such emulsified oils. In a preferred process the emulsified non-silicone oil may be produced via a catastrophic inversion process using a high shear homogeniser (for example, an Ultra Turrax T25 Basic S2 with a S25N-10G dispersing tool, from IKA, Germany). The emulsion may be formed by dropwise addition of water to a mixture of oil and surfactant under shear at a rate of, for example, 0.5 ml per minute prior to the inversion point.

The emulsifying surfactant(s) may be nonionic, cationic, or a mixture of nonionic and cationic and the inclusion level of the total surfactant amount may typically range from 0.1% to 2% by weight of the emulsion. Suitable nonionic surfactants will be known to those skilled in the art and may include for example Lutensol XP-79 (ex BASF). Suitable cationic surfactants will be known to a person skilled in the art and may include for example cetrimmonium chloride.

### Silicone free

The compositions of the invention are free from silicone. In the context of the invention, by free from is meant having less than 0.4 weight %, more preferably less than 0.1 weight %, even more preferably less than 0.05 weight %, still more preferably less than 0.001 weight %, yet preferably less than 0.0001 weight %, and most preferably 0 weight % of silicone by weight of the total composition.

Preferably, the compositions of the invention are also free from silanes.

### The conditioning gel base

The conditioning base comprises a cationic conditioning surfactant and a fatty alcohol.

The composition according to the invention comprises one or more conditioning surfactants which are cosmetically acceptable and suitable for topical application to the hair.

Suitable conditioning surfactants are selected from cationic surfactants, used singly or in admixture. Examples include quaternary ammonium cationic surfactants corresponding to the following general formula:

[N(R¹)(R²)(R³)(R⁴)]⁺ (X)⁻

in which R¹, R², R³, and R⁴ are each independently selected from (a) an aliphatic group of from 16 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halide, (e.g. chloride, bromide), acetate, citrate, lactate, glycolate, phosphate nitrate, sulphate, and alkylsulphate, for example methosulphate, radicals.

The aliphatic groups can contain, in addition to carbon and hydrogen atoms, ether linkages, and other groups such as amino groups. The aliphatic groups, e.g., those of about 12 carbons, or higher, can be saturated or unsaturated.

Specific examples of such quaternary ammonium cationic surfactants of the above general formula are cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride,didodecyldimethylammonium chloride, dioctadecyldimethylammonium chloride, tallowtrimethylammonium chloride, cocotrimethylammonium chloride, dipalmitoylethyldimethylammonium chloride, PEG-2 oleylammonium chloride and salts of these, where the chloride is replaced by other halide (e.g., bromide), acetate, citrate, lactate, glycolate, phosphate nitrate, sulphate, or alkylsulphate.

In a preferred class of cationic surfactant of the above general formula, R¹ is a C16 to C22 saturated or unsaturated, preferably saturated, alkyl chain and R², R³ and R⁴ are each independently selected from CH3 and CH2CH2OH, preferably CH3.

Specific examples of such preferred quaternary ammonium cationic surfactants are cetyltrimethylammonium chloride (CTAC), behenyltrimethylammonium chloride (BTAC) and mixtures thereof.

Preferably, the quaternary ammonium cationic surfactant has a cation selected from cetyltrimethylammonium and behenyltrimethylammonium.

Alternatively, primary, secondary or tertiary fatty amines may be used in combination with an acid to provide a cationic surfactant suitable for use in the invention. The acid protonates the amine and forms an amine salt *in situ* in the hair care composition. The amine is therefore effectively a non-permanent quaternary ammonium or pseudo-quaternary ammonium cationic surfactant.

Suitable fatty amines of this type include amidoamines of the following general formula:

R¹-C(O)-N(H)-R²-N(R³)(R⁴)

in which R¹ is a fatty acid chain containing from 12 to 22 carbon atoms, R² is an alkylene group containing from one to four carbon atoms, and R³ and R⁴ are each independently, an alkyl group having from one to four carbon atoms.

Specific examples of suitable materials of the above general formula are stearamidopropyldimethylamine, stearamidopropyldiethylamine, stearamidoethyldiethylamine,stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and diethylaminoethylstearamide.

Also useful are dimethylstearamine, dimethylsoyamine, oyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

Particularly preferred is stearamidopropyldimethylamine.

The conditioning surfactant is present in the composition in a concentration of 0.1 to 10%, preferably at least 0.5%, more preferably at least 1%, still more preferably at least 2%, even more preferably at least 3% or even at least 4% but typically not more than 9%, preferably not more than 8%, more preferably not more than 7%, still more preferably not more than 6%, even more preferably not more than 5% by weight of the composition.

### The fatty alcohol

The compositions of the invention comprise a fatty alcohol having a carbon-carbon chain length of from C₈ to C₂₂.

The combined use of fatty alcohols and cationic surfactants in conditioning compositions is preferred because this leads to the formation of a lamellar phase, in which the cationic surfactant is dispersed.

The fatty alcohol comprises from 8 to 22 carbon atoms, preferably 16 to 22, most preferably C16 to C18. Fatty alcohols are typically compounds containing straight chain alkyl groups. Preferably, the alkyl groups are saturated. Examples of preferred fatty alcohols include cetyl alcohol, stearyl alcohol and mixtures thereof. The use of these materials is also advantageous in that they contribute to the overall conditioning properties of compositions for use in the invention.

The level of fatty alcohol in conditioners for use in the invention will generally range from 0.01 to 10%, preferably from 0.1 to 8%, more preferably from 0.2 to 7%, most preferably from 0.3 to 6% by weight of the composition.

The weight ratio of cationic-surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

A preferred conditioner comprises a conditioning gel phase having little or no vesicle content. Such conditioners and methods for making them are described in WO2014/016354, WO2014/016353, WO2012/016352 and WO2014/016351.

Such a conditioning gel phase comprises, by total weight of the composition,
i) from 0.4 to 8 wt % of fatty alcohol having from 8 to 22 carbons,
ii) from 0.1 to 2 wt % of cationic surfactant,
and the composition confers a Draw Mass of from 1 to 250 g, preferably 2 to 100 g, more preferably 2 to 50 g, even more preferably 5 to 40 g and most preferably 5 to 25 g to hair treated with the composition.

Draw Mass is the mass required to draw a hair switch through a comb or brush. Thus the more tangled the hair the greater the mass required to pull the switch through the comb or brush, and the greater the level of condition of the hair, the lower the Draw Mass.

The Draw Mass is the mass required to draw a hair switch, for example of weight 1 to 20 g, length 10 to 30 cm, and width 0.5 to 5 cm through a comb or brush, as measured by first placing the hair switch onto the comb or brush, such that from 5 to 20 cm of hair is left hanging at the glued end of the switch, and then adding weights to the hanging end until the switch falls through the comb or brush.

Preferably, the hair switch is of weight 1 to 20 g, more preferably 2 to 15 g, most preferably from 5 to10 g. Preferably, the hair switch has a length of from 10 to 40 cm, more preferably from 10 to 30 cm, and a width of from 0.5 to 5 cm, more preferably from 1.5 to 4 cm.

Most preferably, the Draw Mass is the mass required to draw a hair switch, for example of weight 10 g, length 20 cm, and width 3 cm through a comb or brush, as measured by first placing the hair switch onto the comb or brush, such that from 20 cm of hair is left hanging at the glued end of the switch, and then adding weights to the hanging end until the switch falls through the comb or brush.

Unless otherwise indicated, ratios, percentages, parts, and the like, referred to herein, are by weight.

Aspects of the invention will now be illustrated by the following examples.

### Examples

### Example 1: Preparation of Compositions for use in the ensuing examples

### Preparation of Compositions 1 - 3 in accordance with the invention and Comparative Composition A.

The following conditioner compositions were prepared:
Compositions 1 - 3: Conditioner comprising a blend of oil with different particulate starches in accordance with the invention
Composition A: Comparative conditioner comprising a blend of oil with non-particulate (gelatinised) starch

In the following table, the oil and starch are present in a blend.

The particle size of the particulate starches were measured using the method described herein.

**Table 1: Composition of hair conditioners 1-3, in accordance with the invention and comparative conditioner A.**

| **Ingredients** | **Tradename** | **Wt% in composition** | | | |
|---|---|---|---|---|---|
| | | **A** | **1** | **2** | **3** |
| Cetearyl alcohol | Ginol^{™} 1618 TA | 4 | 4 | 4 | 4 |
| Behenyl trimethyl ammonium chloride | Genamin^{™} BTLF (70 % active) | 1 | 1 | 1 | 1 |
| Cationic surfactant | Lexamine^{™} S-13 | 1 | 1 | 1 | 1 |
| Acid | Lactic acid | 0.32 | 0.32 | 0.32 | 0.32 |
| Emulsified oil | Silkflo^{™} 366/ Lutensol XP-79 (50 % active) | 2 | 2 | 2 | 2 |
| Oil | Silkflo 366 | 0.5 | 0.5 | 0.5 | 0.5 |
| Starch (particle) | Rice PO4 Natural | - | 0.4 | - | - |
| Starch (particle) | D.S.A.7 | - | - | 0.4 | - |
| Starch (particle) | Reisita^{™} Natural | - | - | - | 0.4 |
| Starch (gelatinised) | Agenajel^{™} 20313 | 1 | - | - | - |
| Chelating agent | EDTA | 0.05 | 0.05 | 0.05 | 0.05 |
| Salt | Sodium Chloride | 0.1 | 0.1 | 0.1 | 0.1 |
| Fragrance | - | 0.5 | 0.5 | 0.5 | 0.5 |
| Preservative | - | 0.06 | 0.06 | 0.06 | 0.06 |
| Water | - | up to 100 | up to 100 | up to 100 | up to 100 |

- Agenajel 20313 is a pre-gelatinised maize hydroxypropyl starch phosphate ex Agrana Starch
- Rice PO4 Natural ex Agrana Starch, having a Dv(50) particle size of 7.66 micrometers
- Reisita Natural starch ex Agrana Starch, having a Dv(50) particle size of 8.00 micrometers
- D.S.A.7 starch particles ex Agrana Starch, having a Dv(50) particle size of 8.39 micrometers
- Silkflo 366: polyalphaolefin oil (dec-1-ene) ex Ineos
- Ginol 1618 TA ex Godrej
- Genamin BTLF ex Clariant

The emulsified oil was produced via a catastrophic inversion process using an Ultra Turrax T25 Basic S2 high shear homogeniser and dispersing tool. Water was added dropwise to a mixture of oil and surfactant under shear in a 100 ml stainless steel beaker, at a rate of 0.5 ml per minute, prior to the inversion point. The resulting composition is given in Table 2.

**Table 2: composition of the emulsified oil**

| **Ingredients** | **Tradename** | **Wt % in composition, by total weight of emulsified oil** |
|---|---|---|
| Oil | Silkflo 366 | 50 |
| Emulsifier | Lutensol XP-79 | 0.5 |
| Water | Water | up to 100 |

The compositions in Table 1 were prepared as follows:
1. Water was added to a suitable vessel, lactic acid was added and the vessel heated to 80 °C.
2. Surfactants and fatty materials were added to a suitable vessel and heated to above the melting point of the fatty materials to form a melt.
3. The melt was combined with the water phase and the resultant mixture mixed until opaque and thick.
4. The heat was then turned off and quench water was added.
5. The mixture was then cooled to below 40°C and the rest of the materials, including the emulsified oil, fragrance, were added.
6. The starch and oil were mixed together to form a blend before being added to the composition.
7. Finally the formulation was mixed at high shear on a Silverson mixer at 5000rpm for 5 minutes.

### Preparation of Examples 4 -6 in accordance with the invention and Comparative

### Examples B-E

Further compositions were prepared, using a different conditioner gel base, for use in the ensuing examples:
Compositions 4 -6: Conditioner comprising a blend of oil with different particulate starches in accordance with the invention
Composition B: Comparative conditioner comprising a blend of oil with non-particulate (gelatinised) starch
Composition C: Comparative composition comprising non-blended non-particulate (gelatinised) starch and oil
Compositions D and E: Comparative compositions comprising non-blended particulate starch and oil

The emulsified oil and oil/starch blend were prepared as detailed above.

For compositions comprising non-blended starch and oil, the oil and starch were added separately.

In the following table, the oil and starch are present in a blend, unless indicated by *.

**Table 3: Composition of hair conditioner 4-6, in accordance with the invention and comparative conditioners B-E.**

| **Ingredient** | **Tradename** | **Amount (Wt %)** | | | | | | |
|---|---|---|---|---|---|---|---|---|
| | | **B** | **C** | **D** | **E** | **4** | **5** | **6** |
| Cetearyl alcohol | Ginol 1618 TA | 3.2 | 3.2 | 3.2 | 3.2 | 3.2 | 3.2 | 3.2 |
| Behenyl trimethyl ammonium chloride | Genamin BTLF (70 % active) | 2.29 | 2.29 | 2.29 | 2.29 | 2.29 | 2.29 | 2.29 |
| Chelating agent | EDTA | 0.05 | 0.05 | 0.05 | 0.05 | 0.05 | 0.05 | 0.05 |
| Emulsified oil | Silkflo 366/Lutensol XP-79 (50 % active) | 2 | 2 | 2 | 2 | 2 | 2 | 2 |
| Oil | Silkflo 366 | 1 | 1* | 1* | 1* | 1 | 1 | 1 |
| Starch (particle) | Rice PO4 Natural | - | - | 1* | - | 1 | - | - |
| Starch (particle) | Resita | - | - | - | 1* | - | 1 | - |
| Starch (particle) | D.S.A.7 | - | - | - | - | - | - | 1 |
| Starch (gelatinised) | AGENAJEL^{™} 20313 | 1 | 1* | - | - | - | - | - |
| Fragrance | - | 0.5 | 0.5 | 0.5 | 0.5 | 0.5 | 0.5 | 0.5 |
| Preservative | - | 0.06 | 0.06 | 0.06 | 0.06 | 0.06 | 0.06 | 0.06 |
| Water | - | to 100 | to 100 | to 100 | to 100 | to 100 | to 100 | to 100 |

| | | | | | | | | |
|---|---|---|---|---|---|---|---|---|
| *non-blended oil and starch | | | | | | | | |

- Agenajel 20313 is a pre-gelatinised maize hydroxypropyl starch phosphate ex Agrana Starch
- Rice PO4 Natural ex Agrana Starch
- Resista Natural starch ex Agrana Starch
- D.S.A.7 starch particles ex Agrana Starch
- Silkflo 366: polyalphaolefin oil (dec-1-ene) ex Ineos
- Ginol 1618 TA ex Godrej
- Genamin BTLF ex Clariant

The compositions in Table 3 were prepared as follows:
1. Water was added to a suitable vessel and heated to 80 °C.
2. Surfactant and fatty materials were added to a suitable vessel and heated to above the melting point of the fatty materials to form a melt.
3. The melt was combined with the water phase and the resultant mixture mixed until opaque and thick.
4. The heat was then turned off and quench water was added.
5. The mixture was then cooled to below 40 °C and the rest of the materials, including the emulsified oil and fragrance, were added.
6. The starch and oil were mixed together to form a blend before being added to the composition.
7. Finally, the formulation was mixed at high shear on a Silverson mixer at 5000rpm for 5 minutes.

### Example 2: Nature of starches in Compositions 1 - 3 in accordance with the invention and Comparative Example A

Compositions 1-3 and A were analysed for the presence of particles. The following method was used.

A polarising optical microscope (Olympus BX51) was used to obtain images of the compositions. A small amount of composition was placed on a microscope glass slide with a cover slip on top. In transmittance mode, a 20X magnification objective was used to capture an image under polarised light, as starch particles are birefringent. 10 images per composition were acquired. The number of particles in each image were counted and the average particle per mm² calculated.

The results are given in Table 4 below:

**Table 4: Average number of starch particles in Compositions 1 - 3 in accordance with the invention and comparative example A**

| **Composition** | **Average particles/mm²** | **standard deviation** |
|---|---|---|
| A | 0 | 0 |
| 1 | 1030 | 59 |
| 2 | 767 | 29 |
| 3 | 835 | 25 |

It will be seen that the starches for use in the present invention remain as particles in the conditioner compositions. Composition A, containing Agenajel 20313 starch, does not contain any particles, as the gelatinised starch has dissolved.

### Example 3: Effect of blending oil and starch on coefficient of friction of treated hair

Compositions 4 and 5, according to the invention and comparatives B-E were used to treat hair.

The hair was treated using the following method:
5 g 10 inch Dark Brown European switches were wetted under a tap. A solution of 14 wt % sodium laureth sulfate with one unit of ethoxylation was applied to the switch (at 0.1 g per gram of hair). The switch was massaged for 30 seconds and then rinsed under the tap for 30 seconds. This was repeated. The wet switches were then detangled using a comb. The conditioner composition (0.2 g per gram hair) was then applied to the hair and massaged for 1 min, before being rinsed for 1 min under a controlled flow of water. The switches were left to dry at room temperature.

The coefficient of friction of the hair following treatment was then analysed using a device based upon a flat plate attached to multiple force transducers, with appropriate signal processing and data acquisition. Examples of suitable devices and are described in Guest S. et al, Journal of Cosmetics, Dermatological Sciences and Applications, Vol. 3, 2013, pp. 66-78 and Guest S. et al, Journal of Texture Studies, Vol. 43, 2012, pp. 77-93, and references therein. The specific device used herein employed a Micro Analog 3 signal processing system (Fylde Electronic Laboratories Ltd., UK), along with Dasylab data acquisition software (National Instruments, USA) and six 10 Newton capacity, SMT1 force transducers (Interface Inc., USA) attached to a glass-balsa panel (Aerospace Composite Products, USA). Data presented herein were collected by an operator running a finger along the surface of a dried, treated hair tress (described above), laid flat upon the device, to allow the recording of friction and load forces and calculation of the resulting friction coefficient.

The results are given in Table 5 below:

**Table 5: Coefficient of friction of hair treated with Compositions 4 and 5 and Comparative compositions B-E**

| | CoF | 95 % confidence interval | 90% confidence interval | Confidence interval at which blended is significantly lower than non-blended |
|---|---|---|---|---|
| 4 - PO4 blended | 0.15 | 0.002 | 0.004 | - |
| D - non-blended | 0.19 | 0.007 | 0.011 | 95 |
| 5 - Reisita blend | 0.19 | 0.006 | 0.004 | - |
| E- non-blended | 0.20 | 0.01 | 0.008 | 90 |
| B - Agenajel blended | 0.21 | 0.017 | 0.016 | - |
| C - Agenajel non-blended | 0.21 | 0.02 | 0.012 | n.s.d. |

The results show that with the compositions according to the invention (Example 4 and 5), which comprise a blend of oil and starch, a significantly lower coefficient of friction is obtained than for compositions outside the invention comprising the same oil and starch but in non-blended form.

### Example 4: Rheological properties of Example 4 & 6 in accordance with the invention and Comparative Example B

The rheological properties of compositions 4 and 6, in accordance with the invention and Comparative composition C were tested by measuring the yield stress. The following method was used and the results are given in Table 6.

The product yield stress was measured in an AR-G2 rheometer, from TA Instruments. The method to measure the yield stress uses a serrated parallel-plate geometry, 40mm in diameter, attached to a suitable rheometer capable of applying oscillations at a constant frequency of 1Hz, and an amplitude sweep in the range of 0.1% to 2000%. The amplitude sweep range is applied at no more than ten points per decade of strain range covered at no more than 4 cycles per amplitude. The instrument should be operated under controlled strain. The geometry's temperature should be set at 25°C by means of, for example, a Peltier-controlled plate, or a recirculating bath. The yield stress is determined by plotting the elastic stress against strain amplitude, and at the peak of the curve, the maximum value is quoted as the yield stress.

**Table 6: Yield stress of compositions 4 and 6 (particulate starches), in accordance with the invention and Comparative composition B (non-particulate starch)**

| | **Yield Stress (Pa)** |
|---|---|
| Comparative B | 101 |
| Example 4 | 113 |
| Example 6 | 104 |

The results show that, surprisingly, the yield stress for Examples 4 & 6, in accordance with the invention, are higher than for the comparative composition B.

## Claims

1. A hair conditioner composition comprising
A) 0.01 to 5 wt %,by weight of the total composition, of a blend comprising
i) starch particles having a Dv(50) particle size of from 1 to 12 microns; and
ii) a hydrophobic non-silicone oil;
in a weight ratio of starch to oil of from 0.5:1 to 1: 0.5;
B) 0.01 to 5 wt % of an emulsified non-silicone oil;
wherein A) and B) are dispersed within:
C) a conditioning gel phase comprising a cationic surfactant and a fatty alcohol; and
wherein the conditioner is free from silicone.

2. A composition as claimed in claim 1, wherein the starch is selected from a rice starch, a quinoa starch, an amaranth starch and mixtures thereof.

3. A composition as claimed in claim 1 or claim 2, wherein the starch has a Dv(50) particle size of from 3 to 9 microns.

4. A composition as claimed in any preceding claim, wherein the hydrophobic non-silicone oil is selected from hydrocarbon oils, fatty ester oil and mixtures thereof.

5. A composition as claimed in claim 4, wherein the hydrophobic non-silicone oil is selected from paraffin oil, mineral oil, polyalphaolefin, squalane, esters with hydrocarbyl chains derived from fatty acids or alcohols and mixtures thereof.

6. A composition as claimed in any preceding claim, wherein the emulsified non-silicone oil is selected from paraffin oil, mineral oil, polyalphaolefin, squalane, esters with hydrocarbyl chains derived from fatty acids or alcohols and mixtures thereof.

7. A composition as claimed in any preceding claim, wherein the cationic conditioning surfactant is a quaternary ammonium cationic surfactant.

8. A composition as claimed in claim 7, wherein the quaternary ammonium cationic surfactant has a cation selected from cetyltrimethylammonium and behenyltrimethylammonium.

9. A composition as claimed in any preceding claim, wherein the fatty alcohol has a carbon-carbon chain length of C8 to C22.

10. A method of treating hair, comprising the step of applying a composition as claimed in any preceding claim, to hair.

11. Use of a blend of particulate starch and non-silicone oil in a silicone free composition as defined in claims 1-9 to provide conditioning benefits to hair.

12. Use as claimed in claim 11, wherein the conditioning benefit is a reduced coefficient of friction compared with hair treated with the same composition comprising a non-particulate starch.

## Patentansprüche

1. Haarpflegezusammensetzung, umfassend
A) 0,01 bis 5 Gew.-%, bezogen auf das Gewicht der Gesamtzusammensetzung, einer Mischung, die
i) Stärkepartikel mit einer Dv(50)-Partikelgröße von 1 bis 12 Mikrometern und
ii) ein hydrophobes Nicht-Silikonöl;
in einem Gewichtsverhältnis von Stärke zu Öl von 0,5:1 bis 1:0,5 umfasst;
B) 0,01 bis 5 Gew.-% eines emulgierten Nicht-Silikonöls;
wobei A) und B) dispergiert sind in:
C) einer pflegenden Gelphase, die ein kationisches Tensid und einen Fettalkohol umfasst; und
wobei das Pflegemittel frei von Silikon ist.

2. Zusammensetzung, wie im Anspruch 1 beansprucht, wobei die Stärke unter einer Reisstärke, einer Quinoastärke, einer Amaranthstärke und Mischungen davon ausgewählt ist.

3. Zusammensetzung, wie im Anspruch 1 oder Anspruch 2 beansprucht, wobei die Stärke eine Dv(50)-Partikelgröße von 3 bis 9 Mikrometern aufweist.

4. Zusammensetzung, wie in einem vorhergehenden Anspruch beansprucht, wobei das hydrophobe Nicht-Silikonöl aus Kohlenwasserstoffölen, Fettesteröl und Mischungen davon ausgewählt ist.

5. Zusammensetzung, wie im Anspruch 4 beansprucht, wobei das hydrophobe Nicht-Silikonöl unter Paraffinöl, Mineralöl, Polyalphaolefin, Squalan, Estern mit Hydrocarbylketten, die von Fettsäuren oder Alkoholen und Mischungen davon abgeleitet sind, ausgewählt ist.

6. Zusammensetzung, wie in einem vorhergehenden Anspruch beansprucht, wobei das emulgierte Nicht-Silikonöl unter Paraffinöl, Mineralöl, Polyalphaolefin, Squalan, Estern mit Hydrocarbylketten, die von Fettsäuren oder Alkoholen und Mischungen davon abgeleitet sind, ausgewählt ist.

7. Zusammensetzung, wie in einem vorhergehenden Anspruch beansprucht, wobei das kationische Pflegetensid ein kationisches Tensid vom quaternären Ammoniumtyp ist.

8. Zusammensetzung, wie im Anspruch 7 beansprucht, wobei das kationische Tensid vom quaternären Ammoniumtyp ein Kation aufweist, das aus Cetyltrimethylammonium und Behenyltrimethylammonium ausgewählt ist.

9. Zusammensetzung, wie in einem vorhergehenden Anspruch beansprucht, wobei der Fettalkohol eine Kohlenstoff-Kohlenstoffkettenlänge von C8 bis C22 aufweist.

10. Verfahren zur Behandlung von Haar, umfassend den Schritt des Auftragens einer Zusammensetzung, wie in einem vorhergehenden Anspruch beansprucht, auf das Haar.

11. Verwendung einer Mischung aus partikelförmiger Stärke und Nicht-Silikonöl in einer Silikon-freien Zusammensetzung, wie in den Ansprüchen 1-9 definiert, um dem Haar eine pflegende Wirkung zu verleihen.

12. Verwendung, wie im Anspruch 11 beansprucht, wobei die pflegende Wirkung einen verringerten Reibungskoeffizienten im Vergleich zu einem Haar darstellt, das mit der gleichen Zusammensetzung behandelt wurde, die eine nichtpartikelförmige Stärke enthält.

## Revendications

1. Composition de conditionneur capillaire comprenant
A) 0,01 à 5 % en poids, en poids de la composition totale, d'un mélange comprenant
i) des particules d'amidon ayant une taille de particule Dv(50) de 1 à 12 microns; et
ii) une huile non silicone hydrophobe;
dans un rapport pondéral de l'amidon à l'huile de 0,5:1 à 1:0,5;
B) 0,01 à 5 % en poids d'une huile non silicone émulsifiée;
dans laquelle A) et B) sont dispersés dans:
C) une phase de gel de conditionnement comprenant un tensioactif cationique et un alcool gras; et dans laquelle le conditionneur est exempt de silicone.

2. Composition selon la revendication 1, dans laquelle l'amidon est choisi parmi un amidon de riz, un amidon de quinoa, un amidon d'amarante et leurs mélanges.

3. Composition selon la revendication 1 ou la revendication 2, dans laquelle l'amidon a une taille de particule Dv(50) de 3 à 9 microns.

4. Composition selon l'une quelconque des revendications précédentes, dans laquelle l'huile non silicone hydrophobe est choisie parmi les huiles hydrocarbonées, les huiles d'esters gras et leurs mélanges.

5. Composition selon la revendication 4, dans laquelle l'huile non silicone hydrophobe est choisie parmi l'huile de paraffine, l'huile minérale, une polyalphaoléfine, le squalane, les esters avec des chaînes hydrocarbyles dérivées d'acides ou d'alcools gras et leurs mélanges.

6. Composition selon l'une quelconque des revendications précédentes, dans laquelle l'huile non silicone émulsifiée est choisie parmi l'huile de paraffine, l'huile minérale, une polyalphaoléfine, le squalane, les esters avec des chaînes hydrocarbyles dérivées d'acides ou d'alcools gras et leurs mélanges.

7. Composition selon l'une quelconque des revendications précédentes, dans laquelle le tensioactif de conditionnement cationique est un tensioactif cationique d'ammonium quaternaire.

8. Composition selon la revendication 7, dans laquelle le tensioactif cationique d'ammonium quaternaire a un cation choisi parmi le cétyltriméthylammonium et le béhényltriméthylammonium.

9. Composition selon l'une quelconque des revendications précédentes, dans laquelle l'alcool gras a une longueur de chaîne carbone-carbone de C8 à C22.

10. Procédé de traitement des cheveux, comprenant l'étape d'application d'une composition selon l'une quelconque des revendications précédentes aux cheveux.

11. Utilisation d'un mélange d'amidon particulaire et d'huile non silicone dans une composition sans silicone comme défini dans les revendications 1 à 9 pour procurer des avantages de conditionnement aux cheveux.

12. Utilisation selon la revendication 11, dans laquelle l'avantage de conditionnement est un coefficient de frottement réduit par rapport aux cheveux traités avec la même composition comprenant un amidon non particulaire.
